# EUROPEAN PATENT SPECIFICATION

(11) **EP 2 861 149 B1**
(45) Date of publication and mention of the grant of the patent: **28.10.2015**
(21) Application number: 14730510.6
(22) Date of filing: 13.06.2014
(51) Int. Cl.: A61B 6/03, A61B 6/00, A61B 19/00, A61B 6/04

(54) **COMPUTED TOMOGRAPHY SYSTEM**
COMPUTERTOMOGRAFIESYSTEM
SYSTÈME DE TOMODENSITOMÉTRIE

(30) Priority: 28.06.2013 EP 13174201
(43) Date of publication of application: 22.04.2015
(73) Proprietor: Koninklijke Philips N.V., 5656 AE Eindhoven (NL)
(72) Inventor: JANSSEN, Erik Johannes Maria, NL-5656 AE Eindhoven (NL)
(74) Representative: Steffen, Thomas
(86) International application number: PCT/EP2014/062299
(87) International publication number: WO 2014/206760

(56) References cited:
- BEHROOZ SHARIFI ET AL: "Towards three-dimensional fusion of infrared guidance measurements for biopsy procedures: Some preliminary results and design considerations", SIGNAL PROCESSING AND COMMUNICATION SYSTEMS (ICSPCS), 2010 4TH INTERNATIONAL CONFERENCE ON, IEEE, 13 December 2010 (2010-12-13), pages 1-5, XP031898721, DOI: 10.1109/ICSPCS.2010.5709650 ISBN: 978-1-4244-7908-5
- FAWAD KHAN M ET AL: "Accuracy of biopsy needle navigation using the Medarpa system-computed tomography reality superimposed on the site of intervention", EUROPEAN RADIOLOGY, SPRINGER, BERLIN, DE, vol. 15, no. 11, 1 November 2005 (2005-11-01), pages 2366-2374, XP019335850, ISSN: 1432-1084, DOI: 10.1007/S00330-005-2708-Y
- FRANK BOVA: "Computer Based Guidance in the Modern Operating Room: A Historical Perspective", IEEE REVIEWS IN BIOMEDICAL ENGINEERING, IEEE, USA, vol. 3, 1 January 2010 (2010-01-01), pages 209-222, XP011491272, ISSN: 1937-3333, DOI: 10.1109/RBME.2010.2089370
- NKENKE E ET AL: "Fusion of computer tomography data and optical 3d images of hte dentition for streak artefact correction in the simulation of orthognathic surgery", DENTO-MAXILLO-FACIAL RADIOLOGY, INT. ASS. DENTO-MAXILLO-FACIAL RADIOLOGY, GOTEBORG, SE, vol. 33, 1 January 2004 (2004-01-01), pages 226-232, XP009096553, ISSN: 0250-832X, DOI: 10.1259/DMFR/27071199

## Description

### FIELD OF THE INVENTION

The invention relates to a computed tomography system and to an interventional system comprising the computed tomography system. The invention relates further to a fusion image generation method and computer program for generating a fusion image.

### BACKGROUND OF THE INVENTION

In computed tomography (CT) guided biopsies a patient is moved into a CT imaging region of a computed tomography image generating unit, wherein the computed tomography image generating unit generates a CT image of the patient within the CT imaging region. After the CT image has been generated, the patient is moved out of the computed tomography image generating unit. Then, a physician plans a needle path, along which a needle should be inserted into the patient during the biopsy, based on the generated CT image by using a graphical user interface. In particular, a needle path from an entry point on the patient's skin to a target region within the patient is planned. The physician then needs to estimate the approximate entry point on the patient's skin based on the planned needle path, whereafter the physician can insert the needle into the patient at the approximate entry point over a small distance. The patient is then moved again into the computed tomography image generating unit for generating a further CT image, in order to compare the real position and orientation of the needle shown in the further CT image with the planned needle path. After that the patient is again moved out of the computed tomography image generating unit, and, if the position and orientation of the needle corresponds to the planned needle path, the needle is forwarded and, if, the position and/or the orientation of the needle does not correspond to the planned needle path, the position and/or orientation, respectively, of the needle is corrected. The steps of moving the patient into the computed tomography image generating unit, generating a further CT image for determining the actual position and orientation of the needle, comparing the actual position and orientation of the needle with the planned needle path, and forwarding the needle or correcting the position and/or orientation of the needle are performed, until the needle has reached the target region.

This CT-guided biopsy requires a lot of movements of the patient into and out of the CT imaging region and a lot of CT scans, i.e. a relatively high radiation dose.

In Behrooz Sharifi et.al., 4th International Conference on Signal Processing and Communication Systems (ICSPCS), 2010, IEEE, p1-5, a system of a digital infrared sensitive camera and a high intensity infrared illuminator was used to track infrared reflective tape on a coaxial biopsy needle during a step-wise procedure inserting the needle in a patient followed by CT imaging after each insertion step, whereby the CT image and the actual needle position are combined to show a desired needle insertion angle.

### SUMMARY OF THE INVENTION

It is an object of the present invention to provide a CT system which allows for a reduction of the required number of object movements as well as the radiation dose during an interventional procedure guided by the CT system. It is a further object of the present invention to provide an interventional system comprising the CT system and to provide a fusion image generation method and computer program for generating a fusion image, which allow for a reduction of the number of object movements and of the applied radiation dose during an interventional procedure guided by the CT system.

In a first aspect of the present invention a CT system is provided, wherein the CT system comprises:
- a computed tomography image generating unit for generating a CT image of an object within a CT imaging region,
- a visible light optical image acquisition unit for acquiring an optical image of the object within an outside region outside of the CT imaging region,
- a movable support element for supporting the object and for moving the supported object from the outside region into the CT imaging region and from the CT imaging region into the outside region over a moving distance,
- a path providing unit for providing a path from a location on an outer surface of the object to a target region within the object based on the generated CT image,
- a spatial relation providing unit for providing a spatial relation between a field of view of the computed tomography image generating unit and a field of view of the optical image acquisition unit by a spatial relation providing unit, and
- an image fusion unit for generating a fusion image, in which the CT image and the optical image are fused and which also shows the provided path, based on the CT image, the optical image, the provided path, the provided spatial relation and the moving distance.

Since the fusion image is a combination of the CT image acquired inside the CT imaging region and of the optical image acquired in the outside region outside of the CT imaging region, wherein this fusion image also shows the provided path, a user can very accurately position and orient an interventional instrument at an entry location on an outer surface of the object and insert the interventional instrument along the provided path, while the object is outside the CT imaging region. This accurate placing of the interventional instrument at the entry location and this accurate insertion of the interventional instrument into the object along the provided path leads to a reduced number of required CT images for ensuring that the interventional instrument is really inserted along the provided path. Thus, the number of movements of the object between the CT imaging region and the outside region and the radiation dose applied to the object can be reduced. The reduced number of required movements of the object from the outside region into the CT imaging region and vice versa also reduces the time needed for the interventional procedure.

The object is a person or an animal, in particular, a part of a person or an animal like the thorax of a person or another part of a person. The optical image acquisition unit is adapted to acquire the optical image by detecting visible light, wherein the optical image acquisition unit can be adapted to acquire one or several optical images. The movable support element is preferentially a movable table carrying the object, in particular, carrying the person or the animal.

The path providing unit may be adapted to provide a user interface allowing a user to input the path relative to the reconstructed CT image and to provide the input path. For instance, the CT image can be shown on a display of the CT system and the user interface may allow the user to draw the path from an entry location on an outer surface of the object to the target region within the object in the CT image, wherein the path providing unit can provide this path. Moreover, the path providing unit can also be adapted to automatically determine the path from the entry location on the outer surface of the object to the target region within the object based on the reconstructed CT image. For instance, the path providing unit can be adapted to automatically detect structures within the object and to determine the path from the entry location to the target region based on the detected structures and predefined rules defining a path within the object based on inner structures.

The computed tomography image generating unit preferentially comprises a bore enclosing the CT imaging region, wherein the outside region is outside the bore. Moreover, the optical image acquisition unit may be further adapted to acquire an optical image of the object within the CT imaging region. The optical image acquisition unit may comprise cameras, wherein some cameras are arranged to cover the CT imaging region and some cameras are arranged to cover the outside region.

The spatial relation providing unit can be a storing unit, in which the spatial relation between the field of view of the computed tomography image generating unit and the field of view of the optical image acquisition unit is stored already and from which this spatial relation can be retrieved for providing the same. However, the spatial relation providing unit can also be adapted to determine the spatial relation during a calibration step. For instance, in a calibration step a calibration element comprising optical markers being detectable in an optical image and CT markers being detectable in a CT image can be used, wherein in use the calibration element extends from the CT imaging region to the outside region, wherein, if the calibration element is arranged in the CT imaging region and the outside region, CT markers are in the CT imaging region and optical markers are in the outside region and wherein marker spatial relations between the optical and CT markers are known. In this case the computed tomography image generating unit may be adapted to generate a calibration CT image of the calibration element in the CT imaging region and the optical image acquisition unit may be adapted to acquire a calibration optical image of the calibration element within the outside region. Moreover, the spatial relation providing unit may be adapted to detect the positions of the optical markers in the calibration optical image and the positions of the CT markers in the CT image and to determine spatial relations between the field of view of the computed tomography image generating unit and the field of view of the optical image acquisition unit based on the determined positions. If the optical image acquisition unit is adapted to acquire an optical image of the object also within the CT imaging region, in the calibration step a calibration element may be used, which comprises optical markers also in the CT imaging region, if the calibration element is arranged in the CT imaging region and the outside region. In this case the optical image acquisition unit is adapted to also acquire a calibration optical image of the calibration element within the CT imaging region and the spatial relation providing unit is adapted to detect the positions of the optical markers in the calibration optical images and the positions of the CT markers in the calibration CT image and to determine the spatial relation between the field of view of the computed tomography image generating unit and the field of view of the optical image acquisition unit based on these determined positions. The calibration element is, for instance, a calibration plate comprising the optical and CT markers. These calibration steps allow for an accurate registration of the computed tomography image generating unit and the optical image acquisition unit with respect to each other, thereby determining an accurate spatial relation between the field of view of the computed tomography image generating unit and the field of view of the optical image acquisition unit.

The computed tomography image generating unit is preferentially adapted to generate a three-dimensional CT image, i.e. a volume image, of the object within the CT imaging region, wherein the path from a location on the outer surface of the object to the target region within the object is provided based on the volume image. The image fusion unit is then preferentially adapted to extract from the three-dimensional CT image a two-dimensional CT image, which is fused with the optical image, i.e. the image fusion unit is preferentially adapted to not fuse the entire generated three-dimensional CT image with the optical image, but to fuse a part of the three-dimensional CT image with the optical image, namely the extracted two-dimensional CT image. The extracted two-dimensional CT image corresponds preferentially to a plane within the object, which completely or partly contains the provided path. For instance, the extracted two-dimensional CT image corresponds to a plane which contains at least a part of the provided path at the target region within the object.

In an embodiment an optical marker is arranged in a fixed relation to the movable support element, wherein the optical image acquisition unit is adapted to acquire a first distance measurement optical image of the optical marker, when the object is in the CT imaging region, and a second distance measurement optical image of the optical marker, when the object is in the outside region, wherein the CT system further comprises a moving distance determination unit for determining the moving distance, wherein the moving distance determination unit is adapted to detect the positions of the optical marker in the first and second distance measurement optical images and to determine the moving distance based on the detected positions. Thus, in this embodiment it is not necessary that the moving distance is known in advance or that the moving distance is provided by the support element. The support element with the object can be moved as desired, without requiring the support element to exactly know the moving distance, because the moving distance can be determined by using the optical markers arranged in the fixed relation to the support element. However, in another embodiment the moving distance may also be predefined or may be provided by the support element. The optical markers can be directly attached to the support element, in particular, to an edge of the support element, which will likely not be covered by the object, when the object is arranged on the support element.

In an embodiment optical markers are attached to the object, wherein the optical image acquisition unit is adapted to acquire motion measurement optical images showing the optical markers at different times, wherein the CT system further comprises an object motion determination unit for determining object motion relative to the movable support element, wherein the object motion determination unit is adapted to detect the positions of the optical markers in the motion measurement optical images and to determine the object motion based on the determined positions. In this embodiment the image fusion unit may be adapted to generate the fusion image based on the CT image, the optical image, the provided path, the provided spatial relation, the moving distance and the determined object motion. This can improve the accuracy of showing the provided path relative to the optical image, which in turn can lead to a further reduction of movements of the support element from the outside region into the CT imaging region and vice versa and further reduce the applied radiation dose.

The optical image acquisition unit is preferentially adapted to acquire an actual time-dependent live optical image of the object within the outside region, wherein the image fusion unit is adapted to generate the fusion image such that the CT image and the actual time-dependent live optical image are fused and the fusion image shows the provided path based on the CT image, the actual time-dependent live optical image, the provided path, the provided spatial relation and the moving distance. The actual time-dependent live optical image can show, for instance, an interventional instrument to be positioned and oriented at an entry location in accordance with the provided path, wherein the user can see whether the actual position and orientation of the interventional instrument corresponds to the provided path in realtime. This can make it even easier for the user to accurately position and orient the interventional element in accordance with the provided path, which in turn may further reduce the number of required movements of the support element with the object from the outside region into the CT imaging region and vice versa and may further reduce the applied radiation dose.

In a further aspect of the present invention an interventional system comprising a CT system as defined in claim 1 and an interventional instrument to be moved along a path provided by the path providing unit is presented, wherein the
- optical image acquisition unit is adapted to acquire the optical image of the object in the outside region, while the interventional instrument is placed on the object such that the optical image also shows the interventional instrument,
- the image fusion unit is adapted to generate a fusion image, in which the CT image and the optical image are fused and which also shows the provided path and the interventional instrument, based on the CT image, the optical image, the provided path, the provided spatial relation and the moving distance.

The interventional instrument is preferentially a needle or a catheter to be introduced into a person or an animal along the provided path.

In a further aspect of the present invention a fusion image generation method for generating a fusion image is presented, wherein the fusion image generation method comprises:
- generating a CT image of an object within a CT imaging region by a computed tomography image generating unit,
- providing a path from a location on an outer surface of the object to a target region within the object based on the generated CT image by a path providing unit,
- providing a spatial relation between a field of view of the computed tomography image generating unit and a field of view of the optical image acquisition unit by a spatial relation providing unit, and
- acquiring an optical image of the object within an outside region outside of the CT imaging region by an optical image acquisition unit, after the object has been moved from the CT imaging region to the outside region,
- generating a fusion image, in which the CT image and the optical image are fused and which also shows the provided path, based on the CT image, the optical image, the provided path, the provided spatial relation and the moving distance by image fusion unit.

In a further aspect of the present invention a fusion image generation computer program comprising program code means for causing a CT system as defined in claim 1 to carry out the steps of the fusion image generation method as defined in claim 13 is presented, when the computer program is run on a computer controlling the CT system.

It shall be understood that the CT system of claim 1, the interventional system of claim 12, the fusion image generation method of claim 13, and the fusion image generation computer program of claim 14 have similar and/or identical preferred embodiments, in particular, as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the drawings:
Fig. 1 shows schematically and exemplarily a side view of an embodiment of an interventional system comprising a CT system and an interventional instrument in a first situation, in which an object is arranged within a CT imaging region,
Fig. 2 shows schematically and exemplarily a front view of the CT system shown in Fig. 1,
Fig. 3 shows schematically and exemplarily a side view of the interventional system shown in Fig. 1 in a situation, in which the object is arranged in an outside region outside of the CT imaging region,
Figs. 4 and 5 show fusion images generated by the CT system,
Fig. 6 shows schematically and exemplarily an embodiment of a calibration element for calibrating the CT system, and
Fig. 7 shows a flowchart exemplarily illustrating an embodiment of a fusion image generation method for generating a fusion image.

### DETAILED DESCRIPTION OF EMBODIMENTS

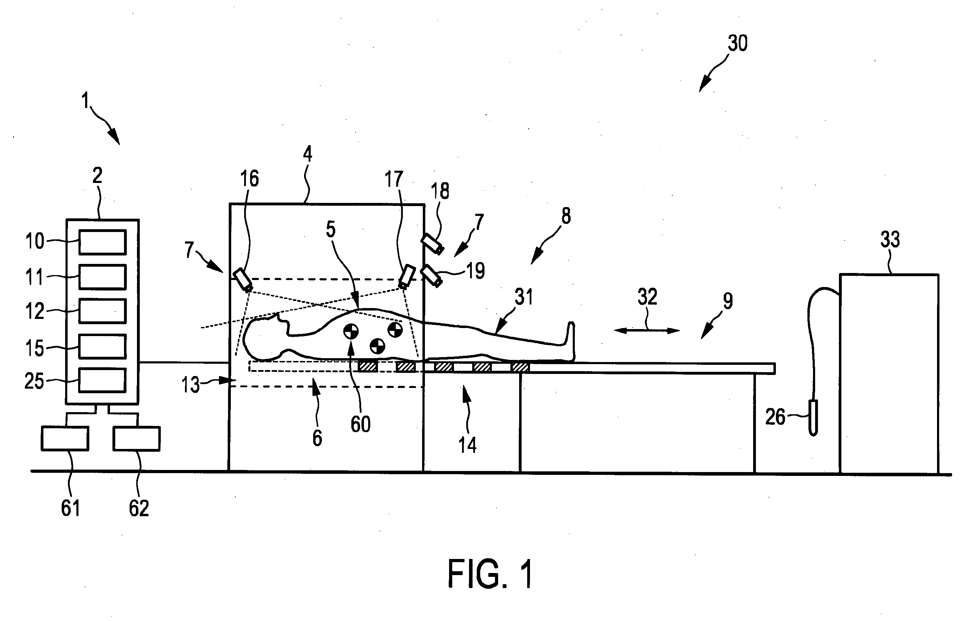

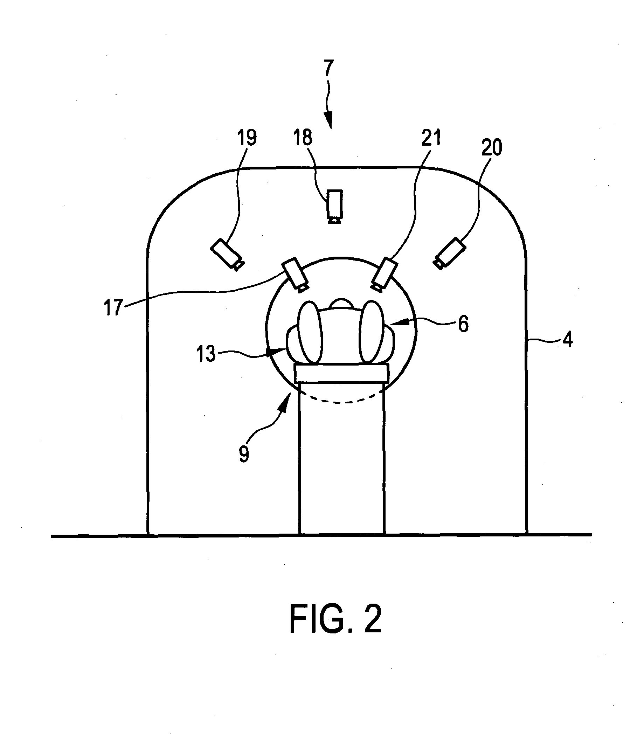

Figs. 1 and 2 schematically and exemplarily show an embodiment of an interventional system 30 comprising a CT system 1, wherein Fig. 1 shows a side view of the entire interventional system 30 and Fig. 2 a front view of the CT system 1 only.

The CT system 1 comprises a computed tomography image generating unit 4 for generating a CT image of an object 5 lying on a movable support element 9. In this embodiment the object 5 is a thorax of a patient 31 and the movable support element 9 is a patient table being movable in the longitudinal direction indicated by the double arrow 32. The computed tomography image generating unit 4 is adapted to generate the CT image of the thorax 5 within a CT imaging region 6 enclosed by a bore 13. In this embodiment the computed tomography image generating unit 4 is adapted to generate a three-dimensional CT image of the object.

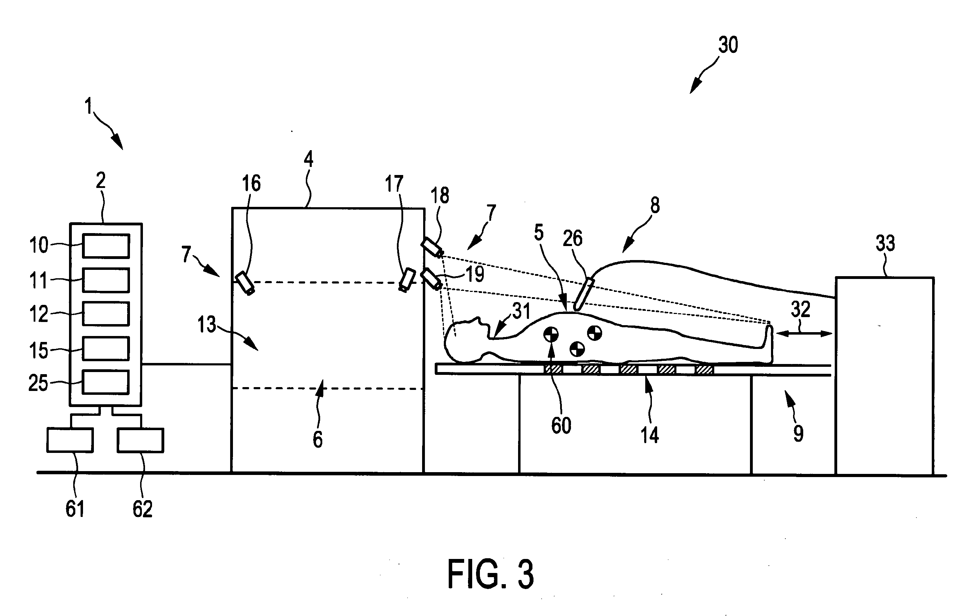

The CT system further comprises an optical image acquisition unit 7 for acquiring an optical image of the thorax 5 within an outside region 8 outside the CT imaging region 6, i.e. outside of the bore 13. The support element 9 is adapted to move the object 5 from the outside region 8 into the CT imaging region 6 and from the CT imaging region 6 into the outside region 8 over a moving distance. In Fig. 1 the object 5 is shown, after it has been moved from the outside region 8 to the inside region 6, whereas in Fig. 3 the object 5 is shown after it has been moved from the CT imaging region 6 into the outside region 8, wherein also Fig. 3 is a side view of the interventional system 30.

Figs. 1 and 3 further show an interventional instrument 26 like a catheter or a needle connected to an interventional instrument control unit 33. The interventional control unit 33 can be adapted, for instance, to provide energy to be applied inside the object 5, to receive sensing signals from the interventional instrument 26 like temperature signals, imaging signals, et cetera and to process these signals for determining a property of the inside of the object, et cetera. The overall system 30 comprising the CT system 1, the interventional instrument 26 and the interventional instrument control unit 33 can therefore be regarded as being an interventional system.

The CT system I further comprises a processing unit 2 with a path providing unit 10 for providing a path from an entry location on an outer surface of the object 5 to a target region within the object 5 based on the generated CT image. The processing unit 2 further comprises a spatial relation providing unit 11 for providing a spatial relation between a field of view of the computed tomography image generating unit 4 and a field of view of the optical image acquisition unit 7 and an image fusion unit 12 for generating a fusion image, in which the CT image and the optical image are fused and which also shows the provided path, based on the CT image, the optical image, the provided path, the provided spatial relation and the moving distance.

The optical image acquisition unit 7 is preferentially adapted to acquire an actual time-pendent live optical image of the object 5 within the outside region 8 in the situation illustrated in Fig. 3, when the interventional instrument 26 is placed on and optionally already inserted into the object 5. The actual time-pendent live optical image shows therefore not only the object 5, but also the interventional instrument 26. If in this situation the image fusion unit 12 generates the fusion image, the fusion image is a fusion of the CT image generated in the situation illustrated in Fig. 1, i.e. when the object 5 was arranged within the CT imaging region 6, and of the actual time-dependent live optical image acquired in the situation illustrated in Fig. 3.

In particular, the image fusion unit 12 is adapted to extract a two-dimensional CT image from the three-dimensional CT image generated by the computed tomography image generating unit 4, wherein the extracted two-dimensional CT image corresponds to a plane completely or partly containing the provided path. The extracted two-dimensional CT image can correspond to a transverse plane, a sagittal plane or a coronal plane of the patient, wherein the respective plane contains at least a part of the provided path, for instance, a part of the path at the target region. However, the extracted two-dimensional CT image can also correspond to a plane which is oriented in another way, for instance, which is oblique with respect to the transverse, sagittal and coronal planes.

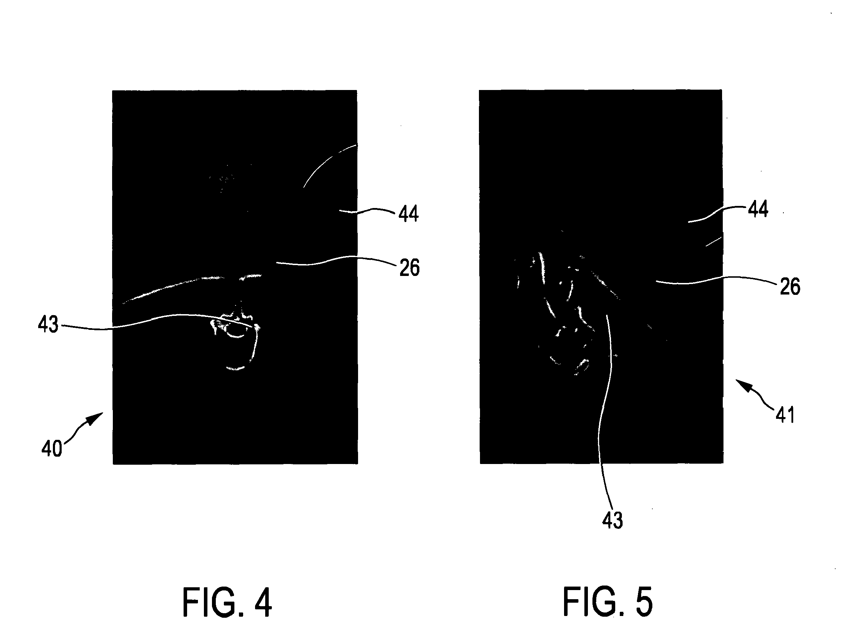

In the fusion image also the provided path from the desired entry location on the outer surface of the object 5 to the target region within the object 5 is indicated by, for instance, a graphical representation, and the actual position and orientation of the interventional instrument 26 outside of the object 5 is shown in the fusion image. Such a fusion image is schematically and exemplarily shown in Fig. 4.

As can be seen in Fig. 4, the fusion image 40 is a fusion of an optical image showing the outside of the object 5 in the outside region 8 and of the extracted two-dimensional CT image, which is extracted from the three-dimensional CT image that had been generated when the object 5 was located in the CT imaging region 6. The fusion image 40 further shows the provided path 43, i.e. a corresponding graphical representation 43, and the actual position and orientation of the interventional instrument 26 held by a hand 44 of a physician. Fig. 5 shows schematically and exemplarily a further fusion image 41, in which an optical image acquired by the optical image acquisition unit 7 in another acquisition direction and a corresponding extracted two-dimensional CT image are fused, wherein also this fusion image further shows the provided path 43, i.e. the corresponding graphical representation 43, and the actual position and orientation of the interventional instrument 26.

The optical image acquisition unit 7 comprises several cameras for acquiring optical images of the outside region 8. In addition, the optical image acquisition unit 7 comprises cameras for acquiring optical images of the object 5 within the CT imaging region 6. In particular, the optical image acquisition unit 7 comprises three cameras 18, 19,20 arranged at the front of the computed tomography image generating unit 4 such that they can acquire optical images of the outside region 8 in front of the computed tomography image generating unit 4, and two pairs of cameras, which are arranged at the two opposing ends of the bore 13 of the computed tomography image generating unit 4 such that they can acquire optical images of the object 5 within the CT imaging region 6. A first pair of cameras is arranged at a first end of the bore 13 and a second pairs of cameras is arranged at an opposing second end of the bore 13. Figs. 1 and 3 show one camera 16 of the first pair of cameras and one camera 17 of the second pair of cameras, and Fig. 2 shows the cameras 17, 21 of the second pair of cameras. In Fig. 1 the lines of sight of the cameras acquiring the optical images of the object 5 within the CT imaging region 6 and in Fig. 3 the lines of sight of the cameras used for acquiring optical images of the object 5 in the outside region 8 are indicated by broken lines.

Optical markers 14 are arranged in a fixed relation to the movable support element 9, wherein the optical image acquisition unit 7 is adapted to acquire a first distance measurement optical image of the optical markers 14, when the object 5 is in the CT imaging region 6 as exemplary shown in Fig. 1, and a second distance measurement optical image of the optical markers 14, when the object 5 is in the outside region 8 as exemplarily shown in Fig. 3, wherein the processing unit 2 further comprises a moving distance determination unit 15 for determining the moving distance, along which the object 5 has been moved from the CT imaging region 6 to the outside region 8. The moving distance determination unit 15 is adapted to detect the positions of the optical markers 14 in the first and second distance measurement optical images and to determine the moving distance based on the detected positions. For detecting the positions of the optical markers in the distance measurement optical images known segmentation algorithms can be used. Moreover, the cameras of the optical image acquisition unit are calibrated such that it is known which position and/or distance within an optical image corresponds to which real position and/or real distance.

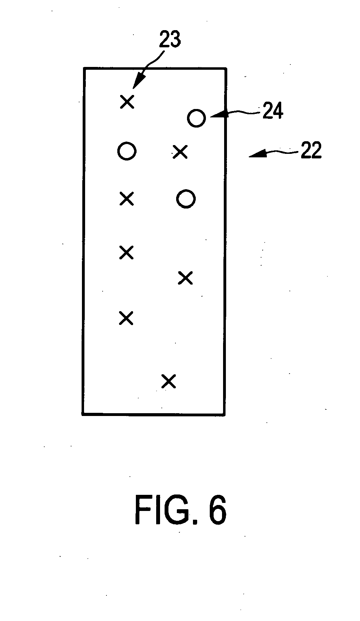

The cameras and also the computed tomography image generating unit 4 may be calibrated in a calibration step by using a calibration element. The calibration element is, for instance, a calibration plate 22 schematically and exemplarily shown in Fig. 6. The calibration plate 22 comprises optical markers 23 being detectable in an optical image and CT markers 24 being detectable in a CT image. Moreover, the calibration plate 22 is dimensioned such that it extends from the CT imaging region 6 to the outside region 8, if the calibration plate 22 is arranged in these regions. Moreover, the optical markers 23 and the CT markers 24 are distributed such that, if the calibration plate 22 is arranged in the CT imaging region 6 and in the outside region 8, the CT markers 24 are in the CT imaging region 6 and the optical markers are in both, the CT imaging region 6 and the outside region 8. In Fig. 6 the upper part of the calibration plate 22 should be arranged in the CT imaging region and the lower part of the calibration plate 22 should be arranged in the outside region 8. The spatial relations between the different markers 23, 24 of the calibration plate 22 are known.

In the calibration step the calibration plate 22 is arranged in the CT imaging region 6 and in the outside region 8 and the computed tomography image generating unit 4 generates a calibration CT image of the calibration plate 22 in the CT imaging region 6. Moreover, the optical image acquisition unit 7 acquires calibration optical images of the calibration plate 22 within the CT imaging region 6 and within the outside region 8. The spatial relation providing unit 11 then detects the positions of the optical markers 23 in the calibration optical images and the positions of the CT markers 24 in the calibration CT image and determines a spatial relation between the field of view of the computed tomography image generating unit 4 and the field of view of the optical image acquisition unit 7 based on the determined positions.

Optical markers 60 are attached to the object 5, wherein the optical image acquisition unit 7 is adapted to acquire motion measurement optical images showing the optical markers 60 at different times, wherein the processing unit 2 further comprises an object motion determination unit 25 for determining object motion relative to the movable support element 9 based on the acquired motion measurement optical images. In particular, the object motion determination unit 25 is adapted to detect positions of the optical markers 60 in the motion measurement optical images and to determine the object motion based on the determined positions. The image fusion unit 12 is preferentially adapted to generate the fusion image also based on the determined object motion, i.e. based on the CT image, which was generated when the object 5 was arranged in the CT imaging region 6 as is exemplarily shown in Fig. 1 and which was used for providing the path within the object 5, on the optical image of the object 5 in the outside region 8, which might be an actual image showing also the interventional instrument 26, on the provided path, on the provided spatial relation, on the moving distance and on the determined object motion.

The path providing unit 10 is adapted to provide a graphical user interface allowing the user to input the path relative to the generated CT image and to provide the input path. The graphical user interface can use an input unit 61 like a keyboard, a computer mouse, et cetera and a display 62. The input unit 61 and the display 62 can also be integrated in a single unit. For instance, the graphical user interface can allow the user to input the path by using a touch screen. In a further embodiment the path providing unit can also be adapted to automatically determine the path based on inner structures of the object 5 shown in the CT image and path detection rules defining a path depending on the detected inner structures of the object 5.

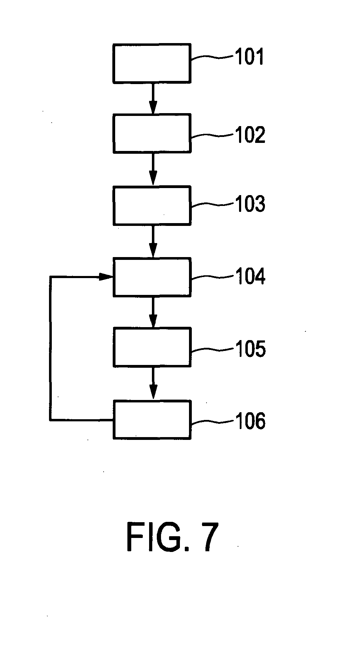

In the following an embodiment of a fusion image generation method for generating a fusion image will exemplarily be described with reference to a flowchart shown in Fig. 7.

After the object 5 has been moved into the CT imaging region 6 as schematically and exemplarily illustrated in Fig. 1, in step 101 the computed tomography image generating unit 4 generates the CT image of the object 5 within the CT imaging region 6. In step 102 the path providing unit 10 provides a path from an entry location on an outer surface of the object 5 to a target region within the object 5 based on the generated CT image. In particular, the path providing unit 10 provides a graphical user interface allowing a user to draw the path in the CT image, wherein the drawn path is provided by the path providing unit 10. However, the path providing unit 10 may also be adapted to automatically or semi-automatically determine the path based on the generated CT image, wherein the determined path is provided. In step 103 a spatial relation between a field of view of the computed tomography image generating unit and a field of view of the optical image acquisition unit is provided by the spatial relation providing unit and, after the object 5 has been moved into the outside region 8 as schematically and exemplarily illustrated in Fig. 3, in step 104 the optical image acquisition unit 7 acquires an optical image of the object 5 within the outside region 8 outside of the CT imaging region 6. This optical image may be an actual image also showing the interventional instrument 26 in its actual position and orientation. In step 105 the image fusion unit 12 generates a fusion image, in which the CT image generated in step 101 and the optical image generated in step 104 are fused and which also shows the provided path, based on the CT image, the optical image, the provided path, the provided spatial relation and the moving distance. In step 106 the fusion image is shown on the display 62.

Steps 104 to 106 are preferentially performed in a loop such that continuously actual optical images are acquired and updated fusion images are generated and shown on the display 62. This allows the physician to arrange the interventional instrument 26 on the object 5 such that the position and orientation corresponds to the provided path, while in realtime the physician can check the correspondence between the actual position and orientation of the interventional instrument 26 and the provided path by looking at the fusion image.

Steps 101 to 106 can also be performed in another order. For instance, step 103 can be performed at any temporal position being before step 105.

By using this fusion image interventional procedures like minimally invasive needle interventions, for instance, biopsies, drainages, ablations, et cetera, can be carried out with reduced applied radiation dose and with less movements of the object 5 from the outside region into the CT imaging region and vice versa, because the interventional instrument may be tracked for a relatively large part of the interventional procedure with the optical image acquisition unit instead of using the computed tomography generating unit, which acquires x-ray projections from the object within the CT imaging region and which reconstructs the CT image based on the acquired x-ray projections.

The optical image acquisition unit comprises a number of optical cameras rigidly attached to the computed tomography image generating unit, i.e. rigidly attached to the CT scanner. The position and orientation of these optical cameras may be such that both, the bore as well as the space in front of the bore, are covered by the field-of-view of the optical cameras. In the embodiment described above with reference to Figs. 1 to 3 four optical cameras cover the space within the bore and three optical cameras cover the space in front of the bore, i.e. four cameras cover the CT imaging region and three cameras cover the outside region.

The positions and orientations of the optical views, i.e. of the field of views, of all optical cameras of the optical image acquisition unit are preferentially calibrated with the position and orientation of the CT image, i.e. of the CT field of view. This calibration is preferentially performed by using the calibration plate, which is large enough to cover both, a surface in the bore and a surface in front of the bore, and which contains optical fiducials, i.e. optical markers visible in the optical images, and x-ray fiducials, i.e. CT markers visible in the CT image. Based on the known relative positions of the x-ray fiducials with respect to the optical fiducials the positions and orientations of the optical views with respect to the CT image can be calculated.

Since the position of the support element is changed, when moving the object out of the bore, in particular, for needle insertion, and the other way around for CT imaging, the positions of the support element need to be accurately known to show the correct provided path, in particular, the correct provided needle path, in the optical images for all positions of the support element. The respective position of the support element can be provided by the support element, if it is correspondingly adapted to accurately deliver its respective position. However, the respective position of the support element can also be provided in another way. For instance, a reproducible patient support-movement system may be created with an additional optical calibration. In this optical calibration the support element is being moved while the actual support element positions with respect to the camera system are registered with the optical cameras using a marker plate which is lying on the support.

By using the CT system described above with reference to Figs. 1 to 3, in particular, comprising the calibrated optical cameras and the calibrated support element, interventional procedures like a CT-guided biopsy can be heavily simplified. For instance, the CT system may allow following workflow for a CT-guided biopsy.

Firstly, the object 5 is moved into the CT, i.e. into the computed tomography image generating unit, by using the longitudinal support element. Then, a CT image of the object in the CT imaging region is generated, whereafter the object is moved out of the CT gantry into the outside region in front of the CT gantry. A path from an entry point on the outside of the object, for instance, on a patient's skin, to a target region, in particular, a target point, within the object is provided. For instance, a physician plans a corresponding needle path based on the generated CT image by using the graphical user interface of the path providing unit. The planned needle path is then visualized in optical images acquired by the optical cameras, which image the space in front of the bore, i.e. which image the outside region, wherein, because the object has been moved out of the CT gantry, the entry point on the object is in the field-of-view of these optical cameras. Since the planned needle path is visualized in these optical images, the physician can position and orient the needle in the right direction and insert it for a couple of centimeters such that critical anatomy cannot be hit. As long as the physician is sure that critical anatomy cannot be hit and the needle is not close to the target region, the physician may continue with inserting the needle. If the physician expects to have inserted the needle close to the target region or to a location close to critical anatomy, the object may be moved again into the CT gantry for generating a low-dose CT image and for checking the actual real needle position and orientation with respect to the planned needle path, whereafter the object can again be moved out of the CT gantry into the outside region in front of the CT gantry. If the checking of the needle position and orientation with respect to the planned needle path showed that the actual position and orientation of the needle is correct, the physician can continue with forwarding the needle into the patient. Otherwise, the physician can correct the position and/or orientation of the needle and then forward the same. The forwarding of the needle under fusion image guidance with a few intermediate CT checking steps can be performed, until the needle has reached the target region. Since a lot of the forwarding and also the positioning of the needle at the entry location and the orientation of the needle at this entry location are performed under fusion image guidance, the total number of CT images and thus the overall time needed for the entire process and the applied radiation dose can be reduced.

The CT system is preferentially adapted to track movements of the object, in particular, to track patient movements. For this purpose a number of optical markers can be applied to the outer surface of the object, in particular, to the skin of the patient. The optical image acquisition unit and the optical markers, i.e. the optical markers 60 described above with reference to Figs. 1 and 3, are preferentially adapted such that four optical markers can be detected by two cameras simultaneously, in order to track the movement of the object. The four individual marker positions can be determined by triangulation, wherein the movement, position and orientation of the object can be determined by using these four actual marker positions.

Although in the embodiment described above with reference to Figs. 1 and 3 the interventional system comprises an interventional instrument and an interventional instrument control unit, in another embodiment the interventional system may just comprise a hand held interventional instrument like a hand held needle, i.e. without the interventional instrument control unit.

Although the optical image acquisition unit described above with reference to Figs. 1 to 3 is adapted to acquire optical images of the object within the CT imaging region and optical images of the object within an outside region outside of the CT imaging region, the optical image acquisition unit can also be adapted to only acquire optical images of the object within the outside region, i.e. the ability to acquire optical images of the object within the CT imaging region is optional. For instance, the optical image acquisition unit may only comprise cameras for imaging an object outside of a bore of a CT system, but not cameras for imaging a region within the bore.

Although in above described embodiments the interventional system is adapted to perform a CT-guided biopsy, in other embodiments the interventional system can be adapted to perform another interventional procedure. For instance, it can be adapted to perform another minimal invasive percutaneous procedure using the CT system, where there is a need to accurately guide a needle or another interventional instrument using CT images.

Although in above described embodiments the object is the thorax, in other embodiment can also be another part of a living being.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the provision of the path, in particular, of the planned needle path, the generation of the fusion image, the determination of the movement of the object relative to the support element, et cetera performed by one or several units or devices can be performed by any other number of units or devices. These procedures and/or the control of the CT system in accordance with the fusion image generation method can be implemented as program code of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a CT system comprising an image fusion unit for generating a fusion image being a fusion of a CT image of an object within a CT imaging region, particularly within a bore of the CT system, and of an optical image of the object, which is generated, after the object has been moved out of the CT imaging region, particularly when the object is located in front of the bore. The fusion image further shows a path, along which an interventional instrument should be moved within the object and which has been provided based on the CT image. By looking at the fusion image a user can accurately move the instrument along the path, without needing to acquire many additional CT images for position checking purposes. This can reduce the radiation dose and time needed for an interventional procedure.

## Claims

1. A computed tomography system comprising:
- a computed tomography image generating unit (4) for generating a computed tomography image of an object (5) within a computed tomography imaging region (6),
- an optical image acquisition unit (7) for acquiring an optical image of the object (5) within an outside region (8) outside of the computed tomography imaging region (6),
- a movable support element (9) for supporting the object (5) and for moving the supported object (5) from the outside region (8) into the computed tomography imaging region (6) and from the computed tomography imaging region (6) into the outside region (8) over a moving distance,
- a path providing unit (10) for providing a path from a location on an outer surface of the object (5) to a target region within the object (5) based on the generated computed tomography image,
- a spatial relation providing unit (11) for providing a spatial relation between a field of view of the computed tomography image generating unit (4) and a field of view of the optical image acquisition unit (7),
- an image fusion unit (12) for generating a fusion image, in which the computed tomography image and the optical image are fused and which also shows the provided path, based on the computed tomography image, the optical image, the provided path, the provided spatial relation and the moving distance,
**characterized in that** the optical image acquisition unit (7) is a visible light optical image acquisition unit.

2. The computed tomography system as defined in claim 1, wherein the optical image acquisition unit (7) is further adapted to acquire an optical image of the object (5) within the computed tomography imaging region (6).

3. The computed tomography system as defined in claim 2, wherein an optical marker (14) is arranged in a fixed relation to the movable support element (9), wherein the optical image acquisition unit (7) is adapted to acquire a first distance measurement optical image of the optical marker (14), when the object (5) is in the computed tomography imaging region (6), and a second distance measurement optical image of the optical marker (14), when the object (5) is in the outside region (8), wherein the computed tomography system further comprises a moving distance determination unit (15) for determining the moving distance, wherein the moving distance determination unit (15) is adapted to detect the positions of the optical marker (14) in the first and second distance measurement optical images and to determine the moving distance based on the detected positions.

4. The computed tomography system as defined in claim 1, wherein in a calibration step a calibration element (22) comprising optical markers (23) being detectable in an optical image and computed tomography markers (24) being detectable in a computed tomography image is used, wherein in use the calibration element (22) extends from the computed tomography imaging region (6) to the outside region (8), wherein, if the calibration element (22) is arranged in the computed tomography imaging region (6) and the outside region (8), computed tomography markers (24) are in the computed tomography imaging region (6) and optical markers (23) are in the outside region (8) and wherein marker spatial relations between the optical and computed tomography markers (23, 24) are known, wherein
- the computed tomography image generating unit (4) is adapted to generate a calibration computed tomography image of the calibration element (22) in the computed tomography imaging region (6),
- the optical image acquisition unit (7) is adapted to acquire a calibration optical image of the calibration element (22) within the outside region (8), and
- the spatial relation providing unit (11) is adapted to detect the positions of the optical markers (23) in the calibration optical image and the positions of the computed tomography markers (24) in the calibration computed tomography image and to determine the spatial relation between the field of view of the computed tomography image generating unit (4) and the field of view of the optical image acquisition unit (7) based on the determined positions.

5. The computed tomography system as defined in claim 4, wherein the optical image acquisition unit (7) is further adapted to acquire an optical image of the object (5) within the computed tomography imaging region (6), wherein, if the calibration element (22) is arranged in the computed tomography imaging region (6) and the outside region (8), optical markers (23) are also in the computed tomography imaging region (6), wherein the optical image acquisition unit (7) is adapted to acquire also a calibration optical image of the calibration element (22) within the computed tomography imaging region (6) and wherein the spatial relation providing unit (11) is adapted to detect the positions of the optical markers (23) in the calibration optical images and the positions of the computed tomography markers (24) in the calibration computed tomography image and to determine the spatial relation between the field of view of the computed tomography image generating unit (4) and the field of view of the optical image acquisition unit (7) based on the determined positions.

6. The computed tomography system as defined in claim 1, wherein optical markers (60) are attached to the object (5), wherein the optical image acquisition unit (7) is adapted to acquire motion measurement optical images showing the optical markers (60) at different times, wherein the computed tomography system (1) further comprises an object motion determination unit (25) for determining object motion relative to the movable support element (9), wherein the object motion determination unit (25) is adapted to detect the positions of the optical markers (60) in the motion measurement optical images and to determine the object motion based on the determined positions.

7. The computed tomography system as defined in claim 6, wherein the image fusion unit (12) is adapted to generate the fusion image based on the computed tomography image, the optical image, the provided path, the provided spatial relation, the moving distance and the determined object motion.

8. The computed tomography system as defined in claim 1, wherein the optical image acquisition unit (7) is adapted to acquire an actual time-dependent live optical image of the object (5) within the outside region (8), wherein the image fusion unit (12) is adapted to generate the fusion image such that the computed tomography image and the actual time-dependent live optical image are fused and the fusion image shows the provided path based on the computed tomography image, the actual time-dependent live optical image, the provided path, the provided spatial relation and the moving distance.

9. The computed tomography system as defined in claim 1, wherein the optical image acquisition unit (7) comprises cameras (16...21) attached to the computed tomography image generating unit (4).

10. The computed tomography system as defined in claim 1, wherein the path providing unit (10) is adapted to provide a user interface allowing a user to input the path relative to the reconstructed computed tomography image and to provide the input path.

11. The computed tomography system as defined in claim 1, wherein the computed tomography image generating unit (4) comprises a bore (13) enclosing the computed tomography imaging region (6), wherein the outside region (8) is outside the bore (13).

12. An interventional system comprising a computed tomography system (1) as defined in claim 1 and an interventional instrument (26) to be moved along a path provided by the path providing unit (10), wherein the
- visible light optical image acquisition unit (7) is adapted to acquire the optical image of the object (5) in the outside region (8), while the interventional instrument (26) is placed on the object (5) such that the optical image also shows the interventional instrument (26),
- the image fusion unit (12) is adapted to generate a fusion image, in which the computed tomography image and the optical image are fused and which also shows the provided path and the interventional instrument (26), based on the computed tomography image, the optical image, the provided path, the provided spatial relation and the moving distance.

13. A fusion image generation method for generating a fusion image, the fusion image generation method comprising:
- generating a computed tomography image of an object (5) within a computed tomography imaging region (6) by a computed tomography image generating unit (4),
- providing a path from a location on an outer surface of the object (5) to a target region within the object (5) based on the generated computed tomography image by a path providing unit (10),
- acquiring an optical image of the object (5) within an outside region (8) outside of the computed tomography imaging region (6) by an optical image acquisition unit (7), after the object has been moved from the computed tomography imaging region (6) to the outside region (8),
- providing a spatial relation between a field of view of the computed tomography image generating unit (4) and a field of view of the optical image acquisition unit (7) by a spatial relation providing unit (11), and
- generating a fusion image, in which the computed tomography image and the optical image are fused and which also shows the provided path, based on the computed tomography image, the optical image, the provided path, the provided spatial relation and the moving distance by an image fusion unit (12),
**characterized in that** the optical image of the object (5) within an outside region (8) outside of the computed tomography imaging region (6) is acquired by a visual light optical image acquisition unit (7)

14. A fusion image generation computer program comprising program code means for causing a computed tomography system as defined in claim 1 to carry out the steps of the fusion image generation method as defined in claim 13, when the computer program is run on a computer controlling the computed tomography system.

## Patentansprüche

1. Computertomographiesystem, das Folgendes umfasst:
- eine Computertomographiebild-Erzeugungseinheit (4) zum Erzeugen eines Computertomographiebilds eines Objekts (5) innerhalb einer Computertomographie-Bildgebungsregion (6),
- eine optische Bilderfassungseinheit (7) zum Erfassen eines optischen Bilds des Objekts (5) innerhalb einer Außenregion (8) außerhalb der Computertomographie-Bildgebungsregion (6),
- ein bewegliches Auflageelement (9) zum Tragen des Objekts (5) und zum Bewegen des darauf liegenden Objekts (5) über einen Verfahrweg aus der Außenregion (8) heraus in die Computertomographie-Bildgebungsregion (6) hinein und aus der Computertomographie-Bildgebungsregion (6) heraus in die Außenregion (8) hinein,
- eine Pfadbereitstellungseinheit (10) zum Bereitstellen eines Pfads von einem Ort auf einer Außenfläche des Objekts (5) zu einer Zielregion innerhalb des Objekts (5) basierend auf dem erzeugten Computertomographiebild,
- eine räumliche Beziehungsbereitstellungseinheit (11) zum Bereitstellen einer räumlichen Beziehung zwischen einem Sichtfeld der Computertomographiebild-Erzeugungseinheit (4) und einem Sichtfeld der optischen Bilderfassungseinheit (7),
- eine Bildverschmelzungseinheit (12) zum Erzeugen eines Fusionsbilds, in dem das Computertomographiebild und das optische Bild verschmolzen sind und das auch den bereitgestellten Pfad zeigt, basierend auf dem Computertomographiebild, dem optischen Bild, dem bereitgestellten Pfad, der bereitgestellten räumlichen Beziehung und dem Verfahrweg,
**dadurch gekennzeichnet, dass** die optische Bilderfassungseinheit (7) eine optische Bilderfassungseinheit für sichtbares Licht ist.

2. Computertomographiesystem nach Anspruch 1, wobei die optische Bilderfassungseinheit (7) weiterhin dafür ausgelegt ist, ein optisches Bild des Objekts (5) innerhalb der Computertomographie-Bildgebungsregion (6) zu erfassen.

3. Computertomographiesystem nach Anspruch 2, wobei eine optische Markierung (14) in einer festen Beziehung zu dem beweglichen Auflageelement (9) vorgesehen ist, wobei die optische Bilderfassungseinheit (7) dafür ausgelegt ist, eine optisches Bild der optischen Markierung (14) bei einem ersten Abstandsmaß zu erfassen, wobei sich das Objekt (5) in der Computertomographie-Bildgebungsregion (6) befindet, und ein optisches Bild der optischen Markierung (14) bei einem zweiten Abstandsmaß zu erfassen, wenn sich das Objekt (5) in der Außenregion (8) befindet, wobei das Computertomographiesystem weiterhin eine Verfahrweg-Ermittlungseinheit (15) zum Ermitteln des Verfahrwegs umfasst, wobei die Verfahrweg-Ermittlungseinheit (15) dafür ausgelegt ist, die Positionen der optischen Markierung (14) in dem optischen Bild bei dem ersten und dem zweiten Abstandsmaß zu detektieren und den Verfahrweg basierend auf den detektierten Positionen zu ermitteln.

4. Computertomographiesystem nach Anspruch 1, wobei in einem Kalibrierschritt ein Kalibrierelement (22) mit optischen Markierungen (23), die in einem optischen Bild detektierbar sind, und mit Computertomographie-Markierungen (24), die in einem Computertomographiebild detektierbar sind, verwendet wird, wobei sich das Kalibrierelement (22) im Betrieb von der Computertomographie-Bildgebungsregion (6) zu der Außenregion (8) erstreckt, wobei, wenn das Kalibrierelement (22) in der Computertomographie-Bildgebungsregion (6) und der Außenregion (8) angeordnet ist, die Computertomographie-Markierungen (24) in der Computertomographie-Bildgebungsregion (6) liegen und die optischen Markierungen (23) in der Außenregion (8) liegen, und wobei die räumlichen Markierungsbeziehungen zwischen den optischen Markierungen und den Computertomographie-Markierungen (23, 24) bekannt sind, wobei
- die Computertomographiebild-Erzeugungseinheit (4) dafür ausgelegt ist, ein Kalibrierungs-Computertomographiebild des Kalibrierelements (22) in der Computertomographie-Bildgebungsregion (6) zu erzeugen,
- die optische Bilderfassungseinheit (7) dafür ausgelegt ist, ein optisches Kalibrierbild des Kalibrierelements (22) innerhalb der Außenregion (8) zu erfassen, und
- die räumliche Beziehungsbereitstellungseinheit (11) dafür ausgelegt ist, die Positionen der optischen Markierungen (23) in dem optischen Kalibrierbild und die Positionen der Computertomographie-Markierungen (24) in dem Computertomographie-Kalibrierbild zu detektieren und die räumliche Beziehung zwischen dem Sichtfeld der Computertomographiebild-Erzeugungseinheit (4) und dem Sichtfeld der optischen Bilderfassungseinheit (7) basierend auf den ermittelten Positionen zu ermitteln.

5. Computertomographiesystem nach Anspruch 4, wobei die optische Bilderfassungseinheit (7) weiterhin dafür ausgelegt ist, ein optisches Bild des Objekts (5) innerhalb der Computertomographie-Bildgebungsregion (6) zu erfassen, wobei, wenn das Kalibrierelement (22) in der Computertomographie-Bildgebungsregion (6) und der Außenregion (8) angeordnet ist, die optischen Markierungen (23) auch in der Computertomographie-Bildgebungsregion (6) liegen, wobei die optische Bilderfassungseinheit (7) dafür ausgelegt ist, auch ein optisches Kalibrierbild des Kalibrierelements (22) innerhalb der Computertomographie-Bildgebungsregion (6) zu erfassen, und wobei die räumliche Beziehungsbereitstellungseinheit (11) dafür ausgelegt ist, die Positionen der optischen Markierungen (23) in den optischen Kalibrierbildern und die Positionen der Computertomographie-Markierungen (24) in dem Computertomographie-Kalibrierbild zu detektieren und die räumliche Beziehung zwischen dem Sichtfeld der Computertomographiebild-Erzeugungseinheit (4) und dem Sichtfeld der optischen Bilderfassungseinheit (7) basierend auf den ermittelten Positionen zu ermitteln.

6. Computertomographiesystem nach Anspruch 1, wobei die optischen Markierungen (60) an dem Objekt (5) angebracht sind, wobei die optische Bilderfassungseinheit (7) dafür ausgelegt ist, optische Bewegungsmaß-Bilder zu erfassen, die die optischen Markierungen (60) zu unterschiedlichen Zeiten zeigen, wobei das Computertomographiesystem (1) weiterhin eine Objektbewegungsermittlungseinheit (25) zum Ermitteln der Objektbewegung relativ zu dem beweglichen Auflageelement (9) umfasst, wobei die Objektbewegungsermittlungseinheit (25) dafür ausgelegt ist, die Positionen der optischen Markierungen (60) in den optischen Bewegungsmaß-Bildern zu detektieren und die Objektbewegung basierend auf den ermittelten Positionen zu ermitteln.

7. Computertomographiesystem nach Anspruch 6, wobei die Bildfusionseinheit (12) dafür ausgelegt ist, das Fusionsbild basierend auf dem Computertomographiebild, dem optischen Bild, dem bereitgestellten Pfad, der bereitgestellten räumlichen Beziehung, dem Verfahrweg und der ermittelten Objektbewegung zu erzeugen.

8. Computertomographiesystem nach Anspruch 1, wobei die optische Bilderfassungseinheit (7) dafür ausgelegt ist, ein tatsächliches zeitabhängiges optisches Live-Bild des Objekts (5) innerhalb der Außenregion (8) zu erfassen, wobei die Bildfusionseinheit (12) dafür ausgelegt ist, das Fusionsbild derartig zu erzeugen, dass das Computertomographiebild und das tatsächliche zeitabhängige optische Live-Bild verschmolzen werden und das Fusionsbild den bereitgestellten Pfad basierend auf dem Computertomographiebild, dem tatsächlichen zeitabhängigen optischen Live-Bild, dem bereitgestellten Pfad, der bereitgestellten räumlichen Beziehung und dem Verfahrweg zeigt.

9. Computertomographiesystem nach Anspruch 1, wobei die optische Bilderfassungseinheit (7) Kameras (16...21) umfasst, die an der Computertomographie-Bilderzeugungseinheit (4) befestigt sind.

10. Computertomographiesystem nach Anspruch 1, wobei die Pfadbereitstellungseinheit (10) dafür ausgelegt ist, eine Benutzeroberfläche bereitzustellen, die es einem Benutzer ermöglicht, den Pfad in Bezug auf das rekonstruierte Computertomographiebild einzugeben, und den Eingabepfad bereitzustellen.

11. Computertomographiesystem nach Anspruch 1, wobei die Computertomographiebild-Erzeugungseinheit (4) einen die Computertomographie-Bildgebungsregion (6) einschließenden Tunnel (13) umfasst, wobei die Außenregion (8) außerhalb des Tunnels (13) liegt.

12. Interventionelles System mit einem Computertomographiesystem (1) nach Anspruch 1 und einem interventionellen Instrument (26), das auf einem durch die Pfadbereitstellungseinheit (10) bereitgestellten Pfad bewegt werden soll, wobei
- die optische Bilderfassungseinheit für sichtbares Licht (7) dafür ausgelegt ist, das optische Bild des Objekts (5) in der Außenregion (8) zu erfassen, während das interventionelle Instrument (26) auf dem Objekt (5) platziert ist, so dass das optische Bild auch das interventionelle Instrument (26) zeigt,
- die Bildfusionseinheit (12) dafür ausgelegt ist, ein Fusionsbild, in dem das Computertomographiebild und das optische Bild verschmolzen sind und das auch den bereitgestellten Pfad und das interventionelle Instrument (26) zeigt, basierend auf dem Computertomographiebild, dem optischen Bild, dem bereitgestellten Pfad, der bereitgestellten räumlichen Beziehung und dem Verfahrweg zu erzeugen.

13. Fusionsbilderzeugungsverfahren zum Erzeugen eines Fusionsbilds, wobei das Fusionsbilderzeugungsverfahren Folgendes umfasst:
- Erzeugen eines Computertomographiebilds eines Objekts (5) innerhalb einer Computertomographie-Bildgebungsregion (6) durch eine Computertomographiebild-Erzeugungseinheit (4),
- Bereitstellen eines Pfads von einem Ort auf einer Außenfläche des Objekts (5) zu einer Zielregion innerhalb des Objekts (5) basierend auf dem erzeugten Computertomographiebild durch eine Pfadbereitstellungseinheit (10),
- Erfassen eines optischen Bilds des Objekts (5) innerhalb einer Außenregion (8) außerhalb der Computertomographie-Bildgebungsregion (6) durch eine optische Bilderfassungseinheit (7), nachdem das Objekt aus der Computertomographie-Bildgebungsregion (6) in die Außenregion (8) bewegt wurde,
- Bereitstellen einer räumlichen Beziehung zwischen einem Sichtfeld der Computertomographiebild-Erzeugungseinheit (4) und einem Sichtfeld der optischen Bilderfassungseinheit (7) durch eine räumliche Beziehungsbereitstellungseinheit (11), und
- Erzeugen eines Fusionsbilds, in dem das Computertomographiebild und das optische Bild verschmolzen sind und das auch den bereitgestellten Pfad zeigt, durch eine Bildverschmelzungseinheit (12) basierend auf dem Computertomographiebild, dem optischen Bild, dem bereitgestellten Pfad, der bereitgestellten räumlichen Beziehung und dem Verfahrweg,
**dadurch gekennzeichnet, dass** das optische Bild des Objekts (5) innerhalb einer Außenregion (8) außerhalb der Computertomographie-Bildgebungsregion (6) durch eine optische Bilderfassungseinheit (7) für sichtbares Licht erfasst wird.

14. Fusionsbilderzeugungscomputerprogramm mit Programmcodemitteln zum Veranlassen eines Computertomographiesystems nach Anspruch 1, die Schritte des Fusionsbilderzeugungsverfahrens nach Anspruch 13 durchzufiihren, wenn das Computerprogramm auf einem Computer ausgeführt wird, der das Computertomographiesystem steuert.

## Revendications

1. Système de tomodensitométrie comprenant :
- une unité de génération d'image de tomodensitométrie (4) permettant de générer une image de tomodensitométrie d'un objet (5) au sein d'une région d'imagerie par tomodensitométrie (6),
- une unité d'acquisition d'image optique (7) destinée à l'acquisition d'une image optique de l'objet (5) au sein d'une région extérieure (8) à l'extérieur de la région d'imagerie par tomodensitométrie (6),
- un élément de support mobile (9) destiné à soutenir l'objet (5) et permettant de déplacer l'objet supporté (5) de la région extérieure (8) vers l'intérieur de la région d'imagerie par tomodensitométrie (6) et de la région d'imagerie par tomodensitométrie (6) vers l'intérieur de la région extérieure (8) sur une distance de déplacement,
- une unité de fourniture de trajectoire (10) pour fournir une trajectoire allant d'un emplacement sur une surface externe de l'objet (5) à une région cible au sein de l'objet (5) sur base d'image de tomodensitométrie générée,
- une unité de fourniture de relation spatiale (11) pour fournir une relation spatiale entre un champ de vision de l'unité de génération d'image de tomodensitométrie (4) et un champ de vision de l'unité d'acquisition d'image optique (7),
- une unité de fusion d'images (12) permettant de générer une image de fusion, dans laquelle l'image de tomodensitométrie et l'image optique sont fusionnées et qui montre également la trajectoire fournie, sur base de l'image de tomodensitométrie, de l'image optique, de la trajectoire fournie, de la relation spatiale fournie et de la distance de déplacement,
**caractérisé en ce que** l'unité d'acquisition d'image optique (7) est une unité d'acquisition d'image optique à lumière visible.

2. Système de tomodensitométrie selon la revendication 1, dans lequel l'unité d'acquisition d'image optique (7) est en outre adaptée pour acquérir une image optique de l'objet (5) au sein de la région d'imagerie par tomodensitométrie (6).

3. Système de tomodensitométrie selon la revendication 2, dans lequel un marqueur optique (14) est disposé dans une relation fixe par rapport à l'élément de support mobile (9), dans lequel l'unité d'acquisition d'image optique (7) est adaptée pour acquérir une première image optique de mesure distance du marqueur optique (14), lorsque l'objet (5) se trouve dans la région d'imagerie par tomodensitométrie (6), et une seconde image optique de mesure distance du marqueur optique (14), lorsque l'objet (5) se trouve dans la région extérieure (8), où le système de tomodensitométrie comprend en outre une unité de détermination de distance de déplacement (15) pour déterminer la distance de déplacement, dans lequel l'unité de détermination de distance de déplacement (15) est adaptée pour détecter les positions du marqueur optique (14) dans les première et seconde images optiques de mesure distance et pour déterminer la distance de déplacement sur base des positions détectées.

4. Système de tomodensitométrie selon la revendication 1, dans lequel, dans une étape d'étalonnage, un élément d'étalonnage (22) comprenant des marqueurs optiques (23) étant détectables dans une image optique et des marqueurs de tomodensitométrie (24) étant détectables dans une image de tomodensitométrie est utilisé, dans lequel, en cours d'utilisation, l'élément d'étalonnage (22) s'étend de la région d'imagerie par tomodensitométrie (6) à la région extérieure (8), dans lequel, si l'élément d'étalonnage (22) est disposé dans la région d'imagerie par tomodensitométrie (6) et la région extérieure (8), les marqueurs de tomodensitométrie (24) sont dans la région d'imagerie par tomodensitométrie (6) et les marqueurs optiques (23) sont dans la région extérieure (8) et dans lequel les relations spatiales de marqueurs entre les marqueurs optiques et de tomodensitométrie (23, 24) sont connues, dans lequel
- l'unité de génération d'image de tomodensitométrie (4) est adaptée pour générer une image de tomodensitométrie d'étalonnage de l'élément d'étalonnage (22) dans la région d'imagerie par tomodensitométrie (6),
- l'unité d'acquisition d'image optique (7) est adaptée pour acquérir une image optique d'étalonnage de l'élément d'étalonnage (22) au sein de la région extérieure (8), et
- l'unité de fourniture de relation spatiale (11) est adaptée pour détecter les positions des marqueurs optiques (23) dans l'image optique d'étalonnage et les positions des marqueurs de tomodensitométrie (24) dans l'image de tomodensitométrie d'étalonnage et pour déterminer la relation spatiale entre le champ de vision de l'unité de génération d'image de tomodensitométrie (4) et le champ de vision de l'unité d'acquisition d'image optique (7) sur base des positions déterminées.

5. Système de tomodensitométrie selon la revendication 4, dans lequel l'unité d'acquisition d'image optique (7) est en outre adaptée pour acquérir une image optique de l'objet (5) au sein de la région d'imagerie par tomodensitométrie (6), dans lequel, si l'élément d'étalonnage (22) est disposé dans la région d'imagerie par tomodensitométrie (6) et la région extérieure (8), les marqueurs optiques (23) sont également dans la région d'imagerie par tomodensitométrie (6), dans lequel l'unité d'acquisition d'image optique (7) est adaptée pour acquérir également une image optique d'étalonnage de l'élément d'étalonnage (22) au sein de la région d'imagerie par tomodensitométrie (6) et dans lequel l'unité de fourniture de relation spatiale (11) est adaptée pour détecter les positions des marqueurs optiques (23) dans les images optiques d'étalonnage et les positions des marqueurs de tomodensitométrie (24) dans l'image de tomodensitométrie d'étalonnage et pour déterminer la relation spatiale entre le champ de vision de l'unité de génération d'image de tomodensitométrie (4) et le champ de vision de l'unité d'acquisition d'image optique (7) sur base des positions déterminées.

6. Système de tomodensitométrie selon la revendication 1, dans lequel les marqueurs optiques (60) sont fixés à l'objet (5), dans lequel l'unité d'acquisition d'image optique (7) est adaptée pour acquérir des images optiques de mesure de mouvement montrant les marqueurs optiques (60) à des moments différents, dans lequel le système de tomodensitométrie (1) comprend en outre une unité de détermination de mouvement d'objet (25) pour déterminer un mouvement d'objet par rapport à l'élément de support mobile (9), dans lequel l'unité de détermination de mouvement d'objet (25) est adaptée pour détecter les positions des marqueurs optiques (60) dans les images optiques de mesure de mouvement et pour déterminer le mouvement d'objet sur base des positions déterminées.

7. Système de tomodensitométrie selon la revendication 6, dans lequel l'unité de fusion d'images (12) est adaptée pour générer l'image de fusion sur base de l'image de tomodensitométrie, de l'image optique, de la trajectoire fournie, de la relation spatiale fournie, de la distance de déplacement et du mouvement d'objet déterminé.

8. Système de tomodensitométrie selon la revendication 1, dans lequel l'unité d'acquisition d'image optique (7) est adaptée pour acquérir une image optique réelle en direct en fonction du temps de l'objet (5) au sein de la région extérieure (8), dans lequel l'unité de fusion d'images (12) est adaptée pour générer l'image de fusion de telle sorte que l'image de tomodensitométrie et l'image optique réelle en direct en fonction du temps sont fusionnées et l'image de fusion montre la trajectoire fournie sur base de l'image de tomodensitométrie, de l'image optique réelle en direct en fonction du temps, de la trajectoire fournie, de la relation spatiale fournie et de la distance de déplacement.

9. Système de tomodensitométrie selon la revendication 1, dans lequel l'unité d'acquisition d'image optique (7) comprend des caméras (16...21) fixées à l'unité de génération d'image de tomodensitométrie (4).

10. Système de tomodensitométrie selon la revendication 1, dans lequel l'unité de fourniture de trajectoire (10) est adaptée pour fournir une interface utilisateur permettant à un utilisateur de saisir la trajectoire par rapport à l'image de tomodensitométrie reconstruite et de fournir la trajectoire saisie.

11. Système de tomodensitométrie selon la revendication 1, dans lequel l'unité de génération d'image de tomodensitométrie (4) comprend un perçage (13) enfermant la région d'imagerie par tomodensitométrie (6), dans lequel la région extérieure (8) est à l'extérieur du perçage (13).

12. Système d'intervention comprenant un système de tomodensitométrie (1) selon la revendication 1 et un instrument d'intervention (26) destiné à être déplacé le long d'une trajectoire fournie par l'unité de fourniture de trajectoire (10), dans lequel
- l'unité d'acquisition d'image optique en lumière visible (7) est adaptée pour acquérir l'image optique de l'objet (5) dans la région extérieure (8), tandis que l'instrument d'intervention (26) est placé sur l'objet (5) de telle sorte que l'image optique montre également l'instrument d'intervention (26),
- l'unité de fusion d'images (12) est adaptée pour générer une image de fusion, dans laquelle l'image de tomodensitométrie et l'image optique sont fusionnées et qui montre également la trajectoire fournie et l'instrument d'intervention (26), sur base de l'image de tomodensitométrie, de l'image optique, de la trajectoire fournie, de la relation spatiale fournie et de la distance de déplacement.

13. Procédé de génération d'image de fusion permettant de générer une image de fusion, le procédé de génération d'image de fusion comprenant :
- la génération d'une image de tomodensitométrie d'un objet (5) au sein d'une région d'imagerie par tomodensitométrie (6) par une unité de génération d'image de tomodensitométrie (4),
- la fourniture d'une trajectoire allant d'un emplacement sur une surface externe de l'objet (5) à une région cible au sein de l'objet (5) sur base de l'image de tomodensitométrie générée par une unité de fourniture de trajectoire (10),
- l'acquisition d'une image optique de l'objet (5) au sein d'une région extérieure (8) à l'extérieur de la région d'imagerie par tomodensitométrie (6) par une unité d'acquisition d'image optique (7), après que l'objet a été déplacé de la région d'imagerie par tomodensitométrie (6) à la région extérieure (8),
- la fourniture d'une relation spatiale entre un champ de vision de l'unité de génération d'image de tomodensitométrie (4) et un champ de vision de l'unité d'acquisition d'image optique (7) par une unité de fourniture de relation spatiale (11), et
- la génération d'une image de fusion, dans laquelle l'image de tomodensitométrie et l'image optique sont fusionnées et qui montre également la trajectoire fournie, sur base de l'image de tomodensitométrie, de l'image optique, de la trajectoire fournie, de la relation spatiale fournie et de la distance de déplacement par une unité de fusion d'images (12),
**caractérisé en ce que** l'image optique de l'objet (5) au sein d'une région extérieure (8) à l'extérieur de la région d'imagerie par tomodensitométrie (6) est acquise par une unité d'acquisition d'image optique en lumière visible (7).

14. Programme informatique de génération d'image de fusion comprenant un moyen formant code de programme pour amener un système de tomodensitométrie selon la revendication 1 à effectuer les étapes du procédé de génération d'image de fusion selon la revendication 13, lorsque le programme d'ordinateur est exécuté sur un ordinateur commandant le système de tomodensitométrie.
